# EUROPEAN PATENT APPLICATION

(11) **EP 3 978 009 A1**
(43) Date of publication of application: **06.04.2022**
(21) Application number: 20817770.9
(22) Date of filing: 02.06.2020
(51) Int. Cl.: A61K 36/8994, A61P 3/00

(54) **TRADITIONAL CHINESE MEDICINE INCREASE AND DECREASE PRESCRIPTION FOR PREVENTING/TREATING METABOLIC SYNDROME AND COMPLICATIONS THEREOF**

(30) Priority: 03.06.2019 CN 201910477389; 26.05.2020 CN 202010456684
(71) Applicant: He, Rongrong, Beijing 101300 (CN)
(72) Inventor: YANG, Genxi, Shanxi 041602 (CN); HE, Rongrong, Beijing 101300 (CN)
(74) Representative: JD&P Patent Attorneys Joanna Dargiewicz & Partners
(86) International application number: PCT/CN2020/093911
(87) International publication number: WO 2020/244501

(57) **Abstract**

A traditional Chinese medicine (TCM) addition and subtraction prescription for preventing/treating metabolic syndrome and comprises: Poria, Semen Coicis, Rhizoma Dioscoreae, Semen Euryales, and Semen Nelumbinis, and can be used for prevention and/or treatment of various dysmetabolic syndrome and complications, e.g., insulin resistance syndrome, diabetes mellitus, hyperlipemia, fatty liver disease, obesity, atherosclerosis, arterial sclerosis, and hypertension; the components of the TCM addition and subtraction prescription are simple and have no toxic or side effects to human body, without tolerance. The TCM addition and subtraction prescription may be orally administered for a long term, and may be appropriately increased or decreased according to disease condition; it may act to different extents, and would not result in hypoglycemia or hypotension decreased blood glucose or decreased blood pressure below normal range, etc. or toxic effects due to too high dose, and brings good news to patients with metabolic syndrome and its related complications.

## Description

### TECHNICAL FIELD

The Invention is involved with the biopharmaceutical industry, particularly involved with a Traditional Chinese Medicine (TCM) addition and subtraction prescription for prevention and/or treatment of metabolic syndrome and complications, and the products made from the TCM addition and subtraction prescription as raw material; the Invention is also involved with the preparation method for the products.

### BACKGROUD OF THE PRESENT INVENTION

Metabolic syndrome (MS) means a human pathological condition with metabolic disorders of protein, fat, and carbohydrate, etc., and is a group of metabolic disorder syndromes and is a risk factor leading to diabetic cardiovascular diseases. It has the following features: ① Multiple metabolic disorders incorporated into one, including obesity, hyperglycemia, hypertension, dyslipidemia, high blood viscosity, hyperuricemia, high fatty liver incidence, and hyperinsulinemia; such metabolic disorders are the pathologic foundation for cardiovascular and cerebrovascular diseases and diabetes mellitus. It is obvious that diabetes mellitus is not an isolated disease, and is an integral part of metabolic syndrome. ② Common pathologic foundation; presently it is mostly considered that their common cause is insulin resistance and hyperinsulinemia resulted from obesity, particularly central obesity. ③ It may result in chance of multiple diseases, e.g., hypertension, coronary heart disease, cerebral stroke, or even some cancers, including breast cancer, endometrial cancer, and prostate cancer related with sex hormones, as well as pancreatic cancer, liver and gall bladder cancer, and colon cancer, etc., of the digestive system. ④ Common prevention and treatment measures; prevention and treatment against a metabolic disorder may also facilitate prevention and treatment of other metabolic disorders (abstracted from the term "metabolic syndrome" of Baidu' encyclopedia).

Glucose metabolic disorders, e.g., diabetes mellitus and diabetic complications, are an important cause for human death. According to the statistical data of the International Diabetes Federation (IDF), global adult patients (20-79 years) with diabetes mellitus was increased from 0.151 billion in 2000 to 0.425 billion in 2017, nearly by 2 folds. It is expected that, by 2045, patients with diabetes mellitus may reach 0.629 billion. Our country is a big country for diabetes mellitus. According to the latest map for diabetes mellitus, issued by IDF in 2017, the diabetic population in China reached 0.114 billion, ranking first in the world. Patients with type II diabetes mellitus accounted for 90%. With increased course of disease, patients with diabetes mellitus may easily develop macrovascular and microvascular injuries, endangering heart, brain, kidney, peripheral nerves, eyes, and feet, etc.

According to the statistics of the World Health Organization, diabetes mellitus has more than 100 complications, and is a known disease with the most complications. In patients diagnosed with diabetes mellitus, 57% patients suffer from complications; moreover, with increased course of disease, the ratio of patients with diabetes mellitus + complications is increased gradually (Analysis Report on Incidence Risk Data for Diabetes Mellitus and Complications in China). The various chronic complications diabetes mellitus are important factors influencing survival rate and quality of life of patients with diabetes mellitus; therefore, proactive prevention and treatment of various diabetic complications is pressing.

Diabetic complications have a function for mutual predictions. For example, urine creatinine, urine microalbumin, and urine albumin are risk factors and markers for retinopathy; it may infer influence of the prescription on retinal microangiopathy from those test results. The statistical clinical data from 1414 patients with type II diabetes mellitus by Rani, et al (2011), showed 1 patient with proteinuria per 6 patients in the type II diabetic population. The chance for diabetic oculopathy in subjects with microalbuminuria is approximately 2 times that in subjects without microalbuminuria; meanwhile, when albuminuria occurs concurrently, the risk for diabetic oculopathy in subjects is increased to 6 folds. Meanwhile, some literatures (Estacio, et al, 1998) have demonstrated that, in Hispanic patient population, albuminuria is considered as a strong predictor for diabetic retinopathy (OR = 2.13, 95% CI = 1.34, 3.37, P = 0.0013). In addition to the above indices for mutual predictions, body weight is also a representative index for metabolic syndrome. Body weight loss may reduce the other various indices of metabolic syndrome, including the symptoms of hyperlipoproteinemia, hypertension, fatty liver disease, arterial sclerosis, atherosclerosis, obesity, and primary hypertension, etc. A follow-up trial in 1653 non-obese adult employees demonstrated that, the more 1-year increase of body weight is, the poorer the indices of metabolic syndrome are (Toga, et al, 2016). Moreover, the health examination data from a second trial in 307 Thailand males aged 20-90 years (mean 45 years) and 295 healthy female showed that, obesity is the major part of metabolic syndrome (Pongchaiyakul, et al, 2007).

Hyperlipoproteinemia, i.e., hyperlipemia, is a manifestation of increased level of one or several classes of lipoproteins. Hyperlipemia plays a very important role in occurrence and evolution of atherosclerosis and consequent cardiovascular events, and is one of the main risk factors for coronary artery disease, stroke, and peripheral vascular diseases. The most common complication of metabolic disorders of lipoprotein is atherosclerosis; hypertriglyceridemia and hyperchylomicronemia may be frequently complicated with fatal diseases, e.g., acute pancreatitis.

In addition to diabetes mellitus and hyperlipemia, metabolic syndrome or insulin resistance syndrome are usually complicated with the symptoms of fatty liver disease, arterial sclerosis, atherosclerosis, obesity, and primary hypertension, etc. Fatty liver disease contained in metabolic syndrome or insulin resistance syndrome may evolve into chronic inflammation, hepatic fibrosis, and hepatic cirrhosis; therefore treatment of fatty liver is also very important; moreover, atherosclerosis, arterial sclerosis, and primary hypertension also account for a high ratio of chronic disease morbidity.

From the above, it is obvious that treatment of diabetes mellitus or metabolic syndrome is not aiming at single internal organ, single metabolic pathway, or single target, while the drugs are considered on the whole to restore function of the various organs, and multiple site administration to open up metabolic pathways, so that the human body can restore normal function; meanwhile, a single drug component may easily result in drug resistance problem. Our prescriptions mainly include the following several components, to conduct prevention and repair for metabolic disorders. The components include:
① Semen Coicis (Adlay seed) the dried ripe kernel of Coix lacryma-jobi L. var. mayuen (Roman.) Stapf (Fam. Gramineae). Semen Coicis belongs to the category of edema-alleviating diuretics; enter the spleen, stomach, and lung meridians; remove dampness, promote urination, invigorate the spleen, stop diarrhea, clear paralysis, discharge pus, detoxicate, and resolve a mass.
② Poria is the dried sclerotium of the fungus, Poria cocos (Schw.) Wolf (Fam. Polyporaceae). Poria belongs to the category of edema-alleviating diuretics; enter the heart, lung, and kidney meridians; remove dampness, promote urination, invigorate the spleen, calm the heart, and tranquilize the mind.
③ Rhizoma Dioscoreae (Dioscorea oppositifolia L.) is the dried rhizome of Dioscorea opposita Thunb. (Fam. Dioscoreaceae). Rhizoma Dioscoreae belongs to the category of qi-tonifying drugs; enter the spleen, lung, and kidney meridians; supplement qi, nourish yin, reinforce the spleen, lung, and kidney, and artringe spontaneous emission or leukorrhea.
④ Semen Euryales is the dried kernel of ripe seed of Euryale ferox Salisb. (Fam. Nymphaeaceae). Semen Euryales belongs to the category of essence-securing, urination-reducing, and leukorrhea-astringing drugs; enter the spleen, kidney, and heart meridians; invigorate the spleen, stop diarrhea, astringe leukorrhea, tonify the kidney, arrest spontaneous emission, nourish the heart, and tranquilize the mind.
⑤ Semen Nelumbinis (Nelumbinis Plumula) is the dried ripe seed of Nelumbo nucifera Gaertn. (Fam. Nymphacaceae). Semen Nelumbinis belongs to the category of essence-securing, urination-reducing, and leukorrhea-astringing drugs; enter the spleen, kidney, and heart meridians; invigorate the spleen, stop diarrhea, astringe leukorrhea, tonify the kidney, arrest spontaneous emission, nourish the heart, and tranquilize the mind.
⑥ Endothelium Corneum Gigeriae Galli (Endothelium corneum) is the dried inner wall of the gizzard of Gallus gallus domesticus Brisson (Fam. Phasianidae). Endothelium Corneum Gigeriae Galli belongs to the category of digestive drugs; sweet; enter the spleen, stomach, small intestine, and bladder meridians; invigorate stomach, promote digestion, astringe spontaneous emission, relieve stranguria, and transform calculi.
⑦ Radix Puerariae (Puerarialobata) the dried root of Pueraria lobata (Willd.) Ohwi. Radix Puerariae belongs to the category of diaphoretic drugs; enter the spleen, stomach, and lung meridians; relieve the skin and muscles, abate fever, help produce saliva, slake thirst, promote eruption, invigorate vital function, stop diarrhea, clear and activate the channels and collaterals, and relieve alcoholism.
⑧ Cortex Cinnamomi (Cinnamomum cassia) is the dried stem bark of Cinnamomum cassia Presl (Fam. Lauraceae). Cortex Cinnamomi has the actions of supplement fire of vital gate, reinforce yang, return fire to the origin, disperse cold, relieve pain, and warm meridians. Cortex Cinnamomi is indicated in impotence, uterine cold, chills and pain of the waist and knees, kidney deficiency and consequent asthma, flaring-up of asthenic yang, vertigo, bloodshot eyes, chills and pain of the heart and abdomen, deficiency cold and consequent vomiting or diarrhea, cold hernia and abdominal pain, and dysmenorrhea or amenorrhea.
⑨ Massa Medicata Fermentata (Medicated Leaven) is prepared by the following procedure: flour or bran is mixed and stirred thoroughly with marzipan, rice bean semen phaseoli, and natural juice of fresh Artemisia Annua, fresh XanThium sibiricum, and fresh polygonum flaccidum to an appropriate humid state, prepared into small masses, placed into a basket, covered with Apocynum venetum or Broussonetia papyrifera leaves, incubated for fermentation for 1 week, and removed when Ashbya gossypii hypha are grown, cut into small pieces, and dried in the sun. Use in crude or stir-baked form. Help digestion and militate the stomach. Indications: Food stagnation, abdominal fullness and distension, and low food intake and anorexia.
⑩ Paeonia Lactiflora is the dried root of Paeonia lactiflora Pall. Paeonia Lactiflora belongs to the category of yang-tonifying drugs; enter the liver and spleen meridians; nourish blood, regulate menstruation, astringe yin and suppress sweating, nourish the liver and relieve pain, and repress the liver yang. In the ancient regimens, Radix Paeoniae Alba and Radix Paeoniae Rubra are called collectively Paeonia Lactiflora. Crude Radix Paeoniae Alba is used to nourish yin, replenish blood, and repress the liver; Radix Paeoniae Alba stir-backed with wine is used to regulate the middle warmer and soothe emergency; Radix Paeoniae Alba stir-backed with earth is used to quite the spleen and stop diarrhea. Therefore Paeonia Lactiflora, particular Radix Paeoniae Alba, is a hypoglycemic drug of first choice and may make qi and blood descending and nourish yin blood.

The treatment cost for the above reviewed metabolic syndrome and complications accounts a lot of the national medical insurance resources, and greatly decreases quality of life of the common people; therefore, it will be a top priority in the national medical and pharmaceutical plan and also a good deed for benefits of both the country and the people to find a group of drugs for effective prevention and treatment of such diseases and symptoms.

### SUMMARY OF PRESENT INVENTION

The TCM addition and subtraction prescription disclosed in the Invention is used for prevention or treatment of metabolic syndrome disease and complications, without toxic and side effects, being safe and effective. The inventor demonstrated the following and completed the Invention, by yearly clinical applications and vigorous animal experiments:

The animal experiment demonstrated that the prescription may decrease body weight and decrease blood glucose, and has therapeutic effects in kidney and eyes; meanwhile, in clinical applications, clinical results also demonstrated that the prescription may achieve successful prevention or treatment of metabolic syndrome and complications, including various metabolic disorders and complications, e.g., insulin resistance syndrome, diabetes mellitus and diabetic complications, hyperlipemia, fatty liver disease, obesity, atherosclerosis, arterial sclerosis, and hypertension.

The objective of the Invention is to provide effective products for prevention or treatment of various metabolic disorders and complications, e.g., insulin resistance syndrome, diabetes mellitus, hyperlipemia, fatty liver disease, obesity, atherosclerosis, arterial sclerosis, and hypertension; the products contain the TCM addition and subtraction prescription as main component

To realize the above objective, the Invention provides effective drug combinations for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention provides the preparation method (including the preparation methods for several dosage forms) for such effective drug combinations for prevention and treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides the uses of such effective drug combinations for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides effective combinations of functional foods or health products, for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides the preparation method for such effective combinations of functional foods or health products, for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides uses for such effective combinations of functional foods or health products, for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides effective combinations of veterinary drugs, animal health product, and feed or feed additives, for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides the preparation methods for such effective combinations of veterinary drugs, animal health product, and feed or feed additives, for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides the uses of such effective veterinary drugs, animal health product, and feed or feed additives, for prevention or treatment of various metabolic disorders and complications, e.g., insulin resistance syndrome, diabetes mellitus, hyperlipemia, fatty liver disease, obesity, atherosclerosis, arterial sclerosis, and hypertension.

The Invention also provides effective methods for prevention or treatment of various metabolic disorders and complications, e.g., insulin resistance syndrome, diabetes mellitus, hyperlipemia, fatty liver disease, obesity, atherosclerosis, arterial sclerosis, and hypertension; the methods include the following steps: A combination in a pharmaceutically effective dose is administered to animals with diabetes mellitus and obesity.

The Invention also provides effective methods for prevention or treatment of various metabolic disorders and complications, e.g., insulin resistance syndrome, diabetes mellitus, hyperlipemia, fatty liver disease, obesity, atherosclerosis, arterial sclerosis, and hypertension; the method include the following steps: a combination in a pharmaceutical effective dose is administered to subjects for prevention or to subjects with various metabolic disorders and complications, e.g., insulin resistance syndrome, diabetes mellitus, hyperlipemia, fatty liver disease, obesity, atherosclerosis, arterial sclerosis, and hypertension.

Specifically, the technical plan of the Invention is provided below:
The Invention has disclosed the TCM addition and subtraction prescription for prevention and/or treatment of dysmetabolic syndrome and complications, containing: *Poria,* Semen Coicis, Rhizoma Dioscoreae, Semen Euryales, and Semen *Nelumbinis.*
Preferably, the TCM addition and subtraction prescription contains any one of *Endothelium Corneum Gigeriae Galli,* Cortex *Cinnamomi, Massa Medicata Fermentata,* Radix *Puerariae,* or *Paeonia Lactiflora,* or any combination of such components;
More preferably, the Paeonia Lactiflora may be replaced with any of the plants containing paeoniflorin, albiflorin, hydroxy paeoniflorin, oxypaeoniflorin, benzoyl paeoniflorin, lactiflorin, paeonol, paeonocide, paeonoside, or paeoniflorinin, or any combination of such plants;
More preferably, the *Endothelium Corneum Gigeriae Galli* may be replaced with any of Fructus *Crataegi,* Semen *Raphani,* Fructus *Hordei Germinatus,* Fructus *Setariae Germinatus, Massa Medicata Fermentata,* protease, or other components with digestive or antanemic function, or any combination of such components;
More preferably, protease includes but not limited to pepsin and trypsin;
More preferably, protease includes but not limited to protease hydrolysate, protease polypeptides, or other enzymes or polypeptides with digestive or antanemic function.
Preferably, any component is in a weight of 1-100 g;
More preferably, any component is in a weight of 1-20 g.

In some specific implementation examples of the Invention, each dose of the TCM addition and subtraction prescription contains 10 g each of Rhizoma Dioscoreae, Semen Euryales, *Poria,* Semen *Nelumbinis,* Semen Coicis, Cortex *Cinnamomi,* Radix *Puerariae,* and 15 g of *Endothelium Corneum Gigeriae Galli.*

In some specific implementation examples of the Invention, each dose of the TCM addition and subtraction prescription contains 10 g each of Rhizoma *Dioscoreae,* Semen *Euryales, Poria,* Semen *Nelumbinis,* Semen Coicis, and *Endothelium Corneum Gigeriae Galli.*

Preferably, the TCM addition and subtraction prescription further includes excipients;

More preferably, the excipients include sugar content and dextrin. In some specific implementation examples of the Invention, the sugar content and dextrin are in a mass/mass ratio of 2:1.

It should be understood that, the excipients of the Invention are not limited to sugar content and dextrin; a technician in the field may select any appropriate aid to complete the Invention, which is within the protection range of the Invention.

preferably, the weight ratio of the *Poria,* Semen *Coicis,* Rhizoma *Dioscoreae,* Semen *Euryales,* and Semen *Nelumbinis* is 1:1:1:1:1.

It should be understood that, the *Poria,* Semen *Coicis,* Rhizoma *Dioscoreae,* Semen Euryales, and Semen *Nelumbinis* are in a mass/mass ratio of not limited to 1:1:1:1:1, an technician of the field may select any appropriate ratio to complete the Invention, which is within the protection range of the Invention.

On the second aspect, the Invention has disclosed a product; the product contains the above-mentioned TCM addition and subtraction prescription;

Preferably, the product is a drug product, health product, food, and feed or feed additives.

More preferably, the drug product and health product is for human use.

Preferably, dosage forms of the product include: powder, paste, granules, pills, tablets, capsules, dissolved medicine, soft extract, decoction, or injection.

Preferably, the product includes: pulverized material, water extract, or organic solvent extract of the raw material components, and residues after extraction of the raw material; the pulverized material of the raw material components include active entity of plant components, or a mixture of such active entities.

On the third aspect, the Invention provides a preparation of the above-mentioned product; the preparation method of the powder is: All the components in the product are subject to processing procedure, pulverization procedure, and blending procedure to produce a powder; the processing procedure, pulverization procedure, and blending procedure are in any order.

Preferably, the decoction is prepared by a conventional preparation method, and the preparation method for the soft extract is: After obtained, the decoction is concentrated to produce a soft extract

In some good implementation examples of the Invention, the decoction is heated to evaporate and concentrate into an extract and then prepared to produce a soft extract

More preferably, the decoction is concentrated to produce an extract, oven-dried, and pulverized, and then is subject to any of the following steps:
a) being tableted to produce tablets;
b) preparation to produce capsules, pills, and paste;
c) granulation to produce granules and dissolved medicine.

Preferably, to save cost, the combinations of the veterinary drugs, animal health product, and feed or feed additives may be prepared by a second processing, extraction, or fermentation of the residues of the raw material after extraction, e.g., the dregs of the decoction, after manufacturing extraction of human drugs, human health products, or food, etc.

On the fourth aspect, the Invention has disclosed the following application:
(1) application of the above-mentioned TCM addition and subtraction prescription for prevention and/or treatment of dysmetabolic syndrome and complications;
(2) application of the above-mentioned product for prevention and/or treatment of dysmetabolic syndrome and complications;
(3) application of the above-mentioned method for prevention and/or treatment of dysmetabolic syndrome and complications;
preferably, the dysmetabolic syndrome includes insulin resistance syndrome, diabetes mellitus, hyperlipemia, fatty liver disease, obesity, atherosclerosis, arterial sclerosis, and hypertension.

On the basis of the general knowledge of the field, the above preferable conditions may be combined in any way, which are within the conception and protection range of the Invention.

Compared with the current techniques, the Invention has the following significant advantages and effects:

The Invention has disclosed an effective TCM addition and subtraction prescription for prevention or treatment of various metabolic disorders and complications, e.g., insulin resistance syndrome, diabetes mellitus, hyperlipemia, fatty liver disease, obesity, atherosclerosis, arterial sclerosis, and hypertension; the results of animal experiments and clinical trials have demonstrated that, the drugs of the Invention have the following advantages:
1) the drugs of the Invention may effectively relieve various metabolic disorders and complications, e.g., insulin resistance syndrome, diabetes mellitus, hyperlipemia, fatty liver disease, obesity, atherosclerosis, arterial sclerosis, and hypertension; diabetic complications include relevant complications, e.g., diabetic nephropathy, oculopathy, hepatopathy, and peripheral neuropathy. Compared with other current drugs against diabetes mellitus, the combinations of the Invention have significant synergistic effects in treatment of diabetes mellitus, and can improve significantly the symptoms of diabetes mellitus and complications and also delay evolution of diabetes mellitus, reduce occurrence of complications, and may also prevent, improve, or treat other various indices of metabolic syndrome and complications.
2) compared with the current chemical therapeutic drugs and natural pure TCM products for treatment of metabolic syndrome, the combinations of the Invention are food-derived components, and the components of the addition and subtraction prescription are simple, without toxic and side effects to human body, without tolerance, and with adverse reactions and side effects decreased significantly. The TCM addition and subtraction prescription may be orally administered for a long term, and may be appropriately increased or decreased according the disease condition; it may act to different extents, and would not result in decreased blood glucose or decreased blood pressure or other indices, below normal range, or poisoning effects due to too high dose, and brings good news to patients with metabolic syndrome.
3) the current therapeutic drugs against diabetes mellitus have acceptable therapeutic effects in the initial period of diabetes mellitus; however, with treatment time, an obvious drug resistance problem occurs, and the therapeutic effects against diabetes mellitus are decreased. The TCM combinations of the Invention contain multiple drug ingredients, with multiple effect targets, with better therapeutic effects, may prevent, improve, and treat various indices of patients with metabolic syndrome, increase medication compliance of patients with metabolic syndrome, and increase patients' quality of life.

### DESCRIPTION OF THE DRAWINGS

Figure 1 shows effects on body weight of diabetic animal model.
Figure 2 shows effects on glycosylated hemoglobin HbA1c (%) of diabetic animal model.
Figure 3 shows effects on UTP 24-h urine albumin quantitation (mg/DL) of diabetic animal model.
Figure 4 shows effects on ALB urine albumin (g/L) of diabetic animal model.
Figure 5 shows effects on mALB urine albumin (g/L) of diabetic animal model.
Figure 6 shows the effects on CRE ^{∗} 20 urine creatinine (µmol/L) of diabetic animal model.

### DETAILED DESCRIPTION OF PREFERRED EMBODIMENTS

Hereafter, in combination with the attached figures and implementation examples, the technical plan of the Invention is in detail; however, such description does not consequently restrict the Invention within the range of the described implementation examples.

In the following implementation examples, the experimental methods of which the specific conditions are not indicated are conducted by normal methods and conditions, or selected in accordance with the Product Instructions.

In the text that follows, the Invention is described in detail. The Invention provides effective drug combinations for prevention and/or treatment of various metabolic disorders and complications, e.g., insulin resistance syndrome, diabetes mellitus, hyperlipemia, fatty liver disease, obesity, atherosclerosis, arterial sclerosis, and hypertension; the drug combinations are an addition and subtraction prescription of several drugs.

The Invention also provides effective drug combinations for prevention and/or treatment of various metabolic disorders and complications, e.g., insulin resistance syndrome, diabetes mellitus, hyperlipemia, fatty liver disease, obesity, atherosclerosis, arterial sclerosis, and hypertension; the drug combinations are an addition and subtraction prescription of several drugs, with the extraction method.

The Invention also provides effective drug combinations for prevention and/or treatment of various metabolic disorders and complications, e.g., insulin resistance syndrome, diabetes mellitus, hyperlipemia, fatty liver disease, obesity, atherosclerosis, arterial sclerosis, and hypertension; the drug combinations are an addition and subtraction prescription of several drugs, with uses.

The diabetic complications are preferably combinations of the following complications, but not limited to such combinations: diabetic retinopathy, diabetic cataract, diabetic hepatopathy, diabetic nephropathy, diabetic neuropathy, heart disease, cancer, osteoporosis, atherosclerosis, and Alzheimer disease. Diabetic complications are symptoms evolved from long duration of diabetes mellitus; the drugs in the prescription for treatment of diabetic complications are the same as those for treatment of diabetes mellitus.

The studies for the patent prescription include the following items:

### 1. Preparation Process of the Invention

TCM products are preferably by the preparation method consisting of the following steps, including but not limited to the following steps:
1) Powder products of the prescription
   ① all components in the product are subject to the preparation step; the preparation method may refer to the Pharmacopoeia of the People's Republic of China published by the China Medical Science Press.
   ② the drug components are pulverized.
   ③ according to a ratio, the pulverized materials are compounded and mixed to produce a powder.
   The processing procedure, pulverization procedure, and blending procedure may be in any order. All components in the product are processed.
2) Extraction of effective ingredients of the prescription
   It includes water extraction method and solvent extraction method; in the later period, an extraction of effective ingredients of the product forms, e.g., granules, decoction, medicinal extract, or tablets, may be conducted.
   ① extraction of TCM products by an extraction solvent;
   ② the extract obtained in step ① is cooled and then filtered; as well as
   ③ optionally, the extract, obtained and filtered in step ②, is concentrated and then dried.
   ④ optionally, the dried extract in step ③ is a granular product or is tableted into tablets.

In the above-mentioned method, TCM components in step ① are purchased from the Tongrentang Pharmacy.

The extraction solvents in this text are water, alcohols, or their mixture. The alcohols are preferably C1-C4 low alcohols. The extraction method in the text is one of the acceptable conventional methods in the field, e.g., filtration, cold soak extraction, hot water extraction, reflux extraction, and ultrasonic extraction. In the text, hot water extraction is preferably used. The extraction is preferably 1-5 repeated extractions, more preferably 2 repeated extractions, but not limited to such extractions. The extraction solvent is added to the dried medicinal herbs, preferably in a 1~10 ^{∗} volume of the mixture of the dried medicinal herbs, more preferably 3 ^{∗} volume. The extraction temperature is preferably but not limited to boiling water extraction. The extraction time is preferably but not limited to 1h-4 h.

### 2. Validation of Animals in the Efficacy Trials of the Invention

### 1) Experimental animals

The test animals in the text are vertebrate animals, preferably mammalian animals, more preferably test animals (e.g., mice, rats, rabbits, guinea pigs, hamsters, dogs, and cats), and most preferably apes (e.g., chimpanzees and gorillas).

The test animals in the text are mice, preferably Grade SPF BKS.Cg-Dock7m+/+Leprdb/J mice (referred to as db/db mice for short) for prevention and treatment of type II diabetic animal model.

### 2) Administration dose and administration mode

The drug combinations in the Invention may contain an extract of TCM mixture, or TCM parts, as active components, in a concentration 0.1wt%-99.9wt%. Any pharmaceutically acceptable vehicle, excipient, or diluent may be added to the combinations.

By mixing with a common diluent or excipient, the combinations of the Invention may be prepared into a product for oral or parenteral administration; the diluents or excipients are, e.g., fillers, extenders, adhesives, humectants, disintegrants, and surfactants. Solid products for oral administration are tablets, pills, powder, granules, and capsules. Such solid products may be prepared from mixing medicinal components and one or multiple appropriate excipients; the excipients are, e.g., starch, calcium carbonate, sucrose or lactose, and gelatin. In addition to simple excipients, lubricants may also be used, e.g., magnesium stearate and talc. Such liquid products for oral administration are suspension, solution, emulsion, and syrup; in addition to common simple diluents, e.g., water and liquid paraffin, the above-mentioned products may contain multiple excipients, e.g., humectants, sweeteners, deodorants, and preservatives. Such products for parenteral administration are sterile water solution, water-insoluble excipients, suspension, emulsion, lyophilized products, and suppositories. In addition to active compounds, water-insoluble excipients and suspension may contain propylene glycol, polyethylene glycol, vegetable oil (e.g., olive oil), and injectable esters (e.g., ethyl oleate), etc.. In addition to active compounds, suppositories may contain Witepsol, polyethylene glycol (macrogol), Tween, cacao butter, trilaurin (laurin butter), and gelatin, etc.

Drug combinations of the Invention for oral or parenteral administration. The combinations of the Invention may be administered via topical use, intraperitoneal injection, intrarectal injection, intravenous injection, intramuscular injection, subcutaneous injection, intrauterine injection, or intraventricular injection. More preferably, the combinations may be administered via topical use.

The effective dose of combinations of the Invention may be determined, based on body weight, age, sex, health condition, diet, administration frequency, administration method, excretion, and disease severity. The dose of an extract of TCM combinations extract is 0.01-2000 mg/kg per day, preferably 30-1000 mg/kg. The administration frequency is once per day, or preferably once to 6 times per day.

The combinations of the Invention may be administered alone, or concurrently with other methods, e.g., surgery, radiotherapy, hormone therapy, chemotherapy, and bioregulators.

### 3) Body weight test

A body weight test is conducted for the animals.

### 4) Blood glucose level and glucose tolerance test

Including oral glucose tolerance test, and measurement of fasting blood glucose level, non-fasting blood glucose level (postprandial blood glucose), and glycosylated hemoglobin (HbA1c) concentration. After measurement of the several indices, the data are recorded and are compared with those in the negative control and positive control, and a statistical analysis is conducted.

### ① fasting blood glucose level and oral glucose tolerance test (OGTT)

Fasting blood glucose means blood glucose level under the basal condition, reflecting the basal function of pancreatic islet B cells, and is an important criterion for diagnosis of diabetes mellitus. Oral glucose tolerance test means determination of fasting blood glucose after animals are fasted, and then animals are administered with the drug and administered intragastrically with glucose solution at the corresponding timepoints, and blood glucose is measured prior to intragastric administration of glucose, and 30 min, 60 min and 120 min after intragastric administration of glucose.

### ② non-fasting blood glucose level

The measured value of 2-H postprandial blood glucose test is the most valuable and may reflect reserve function of pancreatic islet B cells. Blood glucose is measured after animals eat feed.

### ③ measurement of glycosylated hemoglobin (HbA1c) concentration

Glycosylated hemoglobin (GHb) is a product hemoglobin in red blood cells, conjugated with saccharides in serum. GHb is formed via slow, persistent, and irreversible glycation; its content is dependent on blood glucose concentration, and exposure duration of blood glucose to hemoglobin, and independent of blood collection time, patients' fasting or non-fasting status, or insulin use or non-use, etc. Therefore, GHb may efficiently reflect blood glucose control in patients with diabetes mellitus in the past 1-2 month.

Test method: after animal are fasted, 20 µL of whole blood is collected and placed into an EP tube containing HbA1c diluent; the content of HbA1c is measured by a full-automatic analyzer. The specific method refers to the Instructions of the Kit

### 5) Diabetic nephropathy test

Including urine biochemical test (urine total protein, urine albumin, urine microalbumin, and urine creatinine).

Overnight urine is collected and placed on crushed ice to test urine total protein, urine albumin, urine microalbumin, and urine creatinine.

### 3. Clinical Validation of the Prescription

Patients complying with the diagnostic criteria for diabetes mellitus/diabetic nephropathy/hypertension, etc., are used and orally administered with the drug for 3-6 months. The effective rate is calculated after oral administration of the prescription.

### 4. Coverage Range of the Invention

1) Drugs for prevention and treatment of metabolic syndrome and complications
   The Invention also provides drug combinations for prevention and/or treatment of metabolic syndrome and complications, preparation methods; the methods include preparation of the drugs, and the steps for administration of a pharmaceutically effective dose of the combinations containing TCM extract or parts as active components, to subjects with metabolic syndrome and complications.
   The metabolic syndrome is preferably combinations consisting of but not limited to the following complications: various metabolic disorders and complications, e.g., insulin resistance syndrome, diabetes mellitus, hyperlipemia, fatty liver disease, obesity, atherosclerosis, arterial sclerosis, and hypertension.
2) Health products or food for prevention and treatment of metabolic syndrome and complications
   As the products of the Invention are all medicine-and-food homology components, the Invention may also consider health products or food for prevention and improvement of metabolic syndrome and complications; the patent includes drug combinations and preparation methods, the methods include preparation of products, and the steps for administration of a pharmaceutically effective dose of the combinations containing TCM extract or parts as active components, to subjects with metabolic syndrome and complications.
   The metabolic syndrome is preferably combinations consisting of but not limited to the following complications: various metabolic disorders and complications, e.g., insulin resistance syndrome, diabetes mellitus, hyperlipemia, fatty liver disease, obesity, atherosclerosis, arterial sclerosis, and hypertension.
   The health functional food of the Invention may also contain multiple corrigents, additives, or excipients, etc. The food may be daily common food forms, e.g., bread, cookies, noodles, or chewsticks, etc.
3) Veterinary drugs, animal health products, or feed additives for prevention and treatment of metabolic syndrome and complications

In addition to human beings, animals may also suffer from metabolic syndrome and complications, including diabetes mellitus.

The Invention also provides veterinary drugs, animal health products, feed and feed additives for prevention and improvement of metabolic syndrome and complications. Animal experiments and clinical trials conducted in the Invention demonstrate efficiently their therapeutic effects against metabolic syndrome and complications. Therefore, the Invention may be efficiently used to manufacture products for prevention and treatment of metabolic syndrome and complications.

Persistent administration of the veterinary drugs, animal health products, and feed and feed additives to pets, poultry, and livestock may prevent occurrence of metabolic syndrome and complications, and can also cure evolved metabolic syndrome. The dose may be a human dose to 6~12 ^{∗} human dose.

The feed additives of the Invention may contain additionally vehicles universally accepted in the field, that are used to pets, poultry, and livestock. In the Invention, the feed additives may be used separately or concurrently with acceptable vehicles and stabilizers; if required, nutrient substances (e.g., vitamins, amino acids, and mineral substances) or other additives (e.g., antioxidants, antibiotics, and antimicrobial drugs) may be added. The feed additives may be prepared into a form of powder, granules, pills, or suspension. When provided to poultry and livestock, the feed additives of the Invention may be administered separately or mixed with the feed for oral administration.

Implementation Example 1: Preparation of TCM Complex
1) Preparation of TCM complex powder
   TCM materials were all purchased from the Tongrentang Pharmacy; the materials were prepared and then compound proportionally, and pulverized by a TCM pulverizer; the granules passed through a steel sieve in a diameter of 160 microns, and the granules that were not sufficiently fine pass again the steel sieve, until granule size complied with requirements.
2) Water extract of TCM complex
   TCM materials were all purchased from the Tongrentang Pharmacy; the materials were prepared and then compound proportionally, added with an excessive amount of cold water, heated to boil and then gently heated to continue boiling by small fire. The solution was decocted for 2 times, and the two decoctions were mixed to produce a TCM water extract
3) Ethanol extract of TCM complex
   TCM materials were all purchased from the Tongrentang Pharmacy; the materials were prepared and then compound proportionally, and pulverized by a TCM pulverizer; the granules were added with 80% ethanol. The prepared sample was charged into the extraction container and was extracted repeatedly under room temperature. The extract was filtered and concentrated in vacuum. The operating temperature was maintained at 40-45 °C, to prevent decomposition and hydrolysis of ingredients.
4) Methanol extract of TCM complex: Methanol was used to replace ethanol, and preparation of the extract was obtained by the same mode as above in 3).

Implementation Example 2: Experimental Validation of TCM Complex
I. Raising of animal model, and administration of the drugs of the Invention

To investigate TCM complex for prevention and treatment of type II diabetes mellitus in animal model, grade SPF BKS.Cg-Dock7m+/+Leprd b/J mice (referred to as db/db mice for short) were used to conduct a hypoglycemic experiment validation. The mice were allowed with free access to feed and water. The mice were assigned into 4 groups (4 mice per group), and were administered intragastrically with the complex, once per day. The experiment included the control group NOR, diabetes mellitus group MET treated in the metformin group (350 mg/kg), diabetes mellitus group RX-6 treated in the low dose (6 mg/kg) of the drug of the Invention, and diabetes mellitus group RX-12 treated in the high dose (12 mg/kg) of the drug of the Invention. The animals of each group were orally administered with the sample to Week 4. Urine and blood were collected 4 week later.

The drugs in the drug groups included *Poria,* Semen Coicis, Rhizoma Dioscoreae, Semen Euryales, Semen *Nelumbinis, Endothelium Corneum Gigeriae Galli,* and *Paeonia Lactiflora;* the preparation method is that, *Poria,* Semen *Coicis,* Rhizoma *Dioscoreae,* Semen *Euryales,* Semen *Nelumbinis, Endothelium Corneum Gigeriae Galli,* and *Paeonia Lactiflora* were prepared and then compounded in a weight ratio of 1:1:1:1:1:1:2 to produce a powder. The obtained powder was diluted in a certain proportion to obtain RX-6 of the low dose (6 mg/kg) drug group, and RX-12 of the high dose (12 mg/kg) drug group.
1. Animal Model
   Species & strain: BKS.Cg-Dock7m+/+Leprd b/J mice (referred to as db/db mice)
   Grade: Grade SPF
   Gender: Female
2. Raising environment
   The animals were raised in a raise box in an appropriate specification, each in a single box. In the Grade SPF animal room, the environmental conditions were controlled at room temperature 20-26 °C, relative humidity 40%-70%, with 12/12-h alternating light and dark cycles.
3. Feed
   The animals were allowed with free access to acceptable mouse feed. A qualified testing institution tested nutrient components (main test components: crude protein, crude fat, crude fiber, water, calcium, total phosphorus, and crude ash) and physicochemical indices (main test indices: arsenic, lead, mercury, cadmium, benzene hexachloride, dichloro-diphenyl-trichloroethane, and aflatoxin B1) of each batch of normal feed. The director of the animal experiment department or designee determined whether there was any contaminant that would influence or interfere with the test results. The test results were evaluated with reference to the national standards GB14924.2-2001 and GB14924.3-2010.
4. Drinking water
   The animals were given purified water, filtered via grade 2 reverse osmosis RO membrane. The animals were allowed with free access to water supplied by water bottle. Water bottles and bottle cork were disinfected by autoclave. Appearance and microbial indices (main test indices: total colony count, odor and taste, visible substance) of the drinking water are tested monthly, toxicological indices (arsenic, lead, mercury, cadmium, and visible substance) including various contaminants were tested yearly; the director of the animal experiment department or designee ensured that the was no contaminant that would influence or interfere with the test results. The analysis results of drinking water were evaluated with reference to the national standards GB5749-2006.
5. Beddings
   The beddings were dedicated trial beddings autoclaved, made of wood shavings material. Contaminants (main test indices: arsenic, lead, mercury, cadmium, benzene hexachloride, dichloro-diphenyl-trichloroethane, and aflatoxin B1) were tested yearly by a qualified institution. Microbial test (main test indices: total colony count, visible substance) is conducted monthly.

### II. Trial Design

### 1. Animal groups and administration dose

The animals were randomly assigned into 4 groups, based on body weight The doses of each group were shown in Table 1 below:

| **Table 1** Groups administered different dose | | |
|---|---|---|
| Group | Administered dose (mg/kg) | Dose concentration (ml/kg) |
| 1. Negative control group (NOR) | - | - |
| 2. Positive control group (MET) | 0.3 | 20 |
| 3. Low dose group (RX-6) of the test article | 6 | 20 |
| 4. High dose group (RX-12) of the test article | 12 | 25 |

### 2. Dosing information

### Administration route: Intragastric administration

Dosing capacity: 10 mL/kg; the dosing capacity for each animal was determined, based on the latest body weight of the animal.

Administration frequency: Once per day, for 4 weeks (the animals were fasted for approximately 4 h prior to administration of the drug).

### 3. General clinical observations

Observation frequency: During the trial, all the animals were observed grossly (once in the morning and once in the afternoon).

Observation contents: including animals' mental state, behaviors, and food intake, etc.

### III. Hypolipemic in the animal experiments of the Invention

Including body weight measuring experiment and the testing experiment for urine indices. After measurement of the indices, the data were recoded and are compared with those in the drug group and negative control group NOR, positive control group MET of the Invention, and a statistical analysis was conducted.

### 1. Measurement of body weight

The animals were weighed once prior to receipt, prior to administration of the first dose, and on the date of the first dose, and then twice per week after administration of the drug.

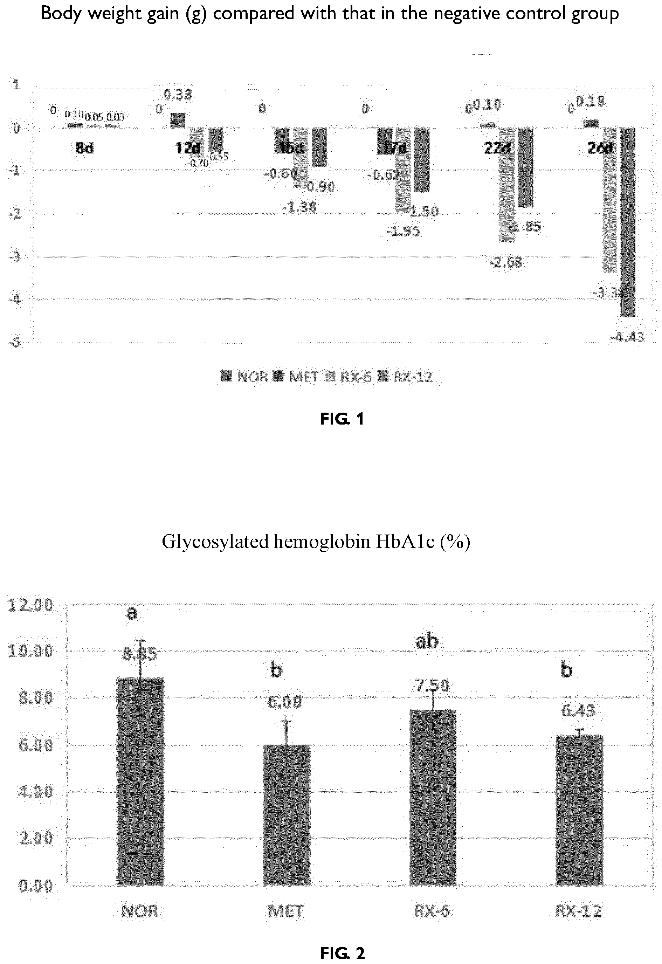

The experimental results (see Figure 1) showed that, with administration time, the low and high dose groups of our Invention had the effects of decreasing the body weight of mice, compared with the negative control group NOR and positive control group MET

The results demonstrated that, high dose of the drug of the Invention may decrease significantly mice body weight, while obesity is exactly the direct incentive for metabolic syndrome. The results of many experiments demonstrated that, body weight is significantly correlated with the indices of metabolic syndrome, including blood pressure and triglyceride (TG), and BMI, etc. (Toga, et al, 2015; Pongchaiyakul, et al, 2007). Through the effects of significantly decreased body weight in animal experiments, it may be speculated that the drugs of our Invention have therapeutic effects against the various indices of metabolic syndrome and complications.

### IV. Hypoglycemic Effects of the Animal Experiments of the Invention

Including measurement of fasting blood glucose level, non-fasting blood glucose level (postprandial blood glucose), and glycosylated hemoglobin (HbA1c) concentration.

After measurement of the several indices, the data were recoded and were compared with those in the negative control group NOR and positive control group MET, and a statistical analysis was conducted.

### 1. Measurement of fasting blood glucose level

After animals were fasted for 4-6 h, blood was collected at tail end to measure fasting blood glucose. Fasting blood glucose was measured for 4 times, i.e.: prior to grouping, and D₂, D₇, and D₁₅ after administration of the drug.

The measured results of fasting blood glucose level showed that, postprandial blood glucose value in the positive control group MET 2 d later after oral administration of the drug was less than that in negative control group NOR; while the blood glucose levels in the high dose group RX₋₁₂ and low dose group RX₋₆ were decreased gradually, and were less than that in the negative control group NOR on D₁₅ after oral administration of the drug (the data were not shown).

### 2. Non-fasting blood glucose level

The animals were fed randomly and then blood was collected at tail end to measure non-fasting blood glucose level. Fasting blood glucose was measured for 6 times, i.e.: prior to grouping, and 24 h, D₄, D₇, D₁₅, D₂₁, and D₂₇ after administration of the drug.

The measured results of non-fasting blood glucose level showed that, the blood glucose level in the positive control group MET was less than that in the positive control on D₄ after oral administration of the drug, while those in the high dose group RX₋₁₂ and low dose group RX₋₆ were less than that in the positive control on D₇ and tended to decrease gradually.

### 3. Measurement of glycosylated hemoglobin (HbA1c) concentration

The concentration was measured on D₂₉ after oral administration of the drug. The animals were fasted for 4-6 h, and then anesthetized by inhalation of isoflurane; 20 L of whole blood was collected from orbital venous plexus and placed into a EP tube containing HbA1c diluent; the content of HbA1c was measured by a full-automatic analyzer. The specific method referred to the Instructions of the Kit For statistical analysis adopts, Duncan 's multiple range test was adopted to conduct a significance analysis for differences between samples of multiple groups (P < 0.05).

According to the data in Figure 2, the glycosylated hemoglobin value in the negative control group NOR was 8.85%, and there were significant differences of glycosylated hemoglobin values between the positive control group MET (6%) and high dose group RX-12 (6.43%), and the negative control group NOR (8.85%); while the glycosylated hemoglobin value in the low dose group RX-6 (7.50%) was decreased by 1.35% than that in the negative control group, and there were no significant differences.

### 4. Summary and Discussion

The experiment of this section was to demonstrate hypoglycemic effects of the drugs of the Invention by measurement of three indices (fasting blood glucose level, non-fasting blood glucose level, and glycosylated hemoglobin). Fasting blood glucose level and non-fasting blood glucose level were measured at different timepoints (fasting blood glucose level: prior to grouping and D₂, D₇, and D₁₅ after administration of the drug; non-fasting blood glucose level: prior to grouping and 24h, D₄, D₇, D₁₅, and D₂₁ after administration of the drug); the fasting blood glucose level on D₁₅ and the non-fasting level on D₇ from the drug of the Invention were less than the negative control value, and tended to decrease gradually; while on D₂₉ after administration of the drug, the glycosylated hemoglobin value (6.43%) in the high dose group RX₋₁₂ was significantly less than negative control group NOR (8.85%).

The results of the hypoglycemic experiment part also demonstrated that, the drugs of the Invention may decrease slowly and significantly blood glucose value in mice.

### V. Test for diabetic nephropathy

Overnight urine was collected from the metabolism cage and was placed on crushed ice to test urine total protein, urine albumin, urine microalbumin, and urine creatinine. The test time was D₂₉ after administration of the drug.

### 1. Quantitative test for UTP 24-h urine protein

Quantitative test for 24 h urine protein (UTP24) is a urine test method in which 24-h discharged total urine is collected to conduct a qualitative test

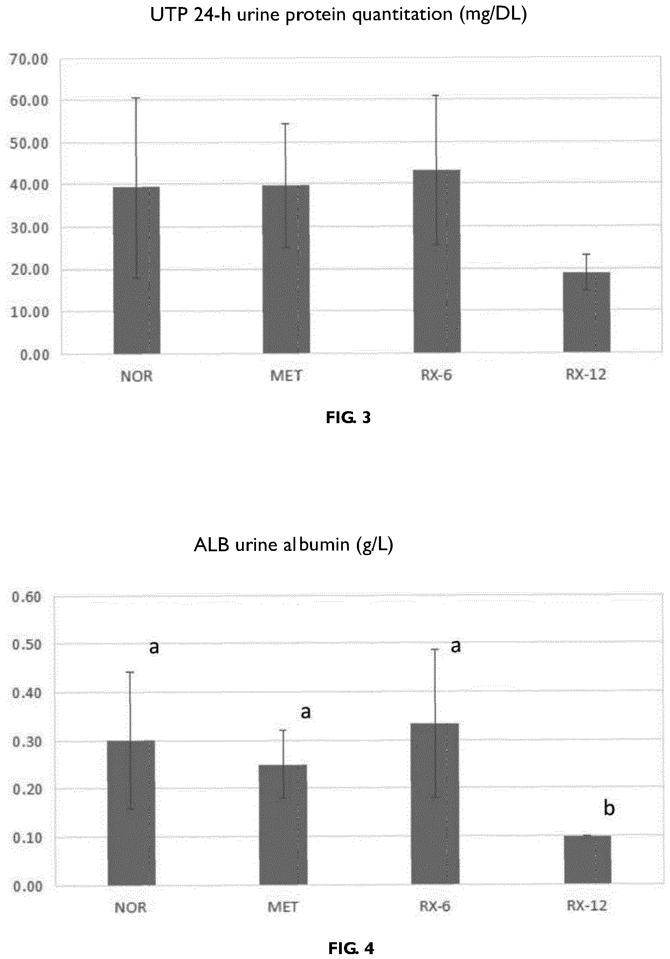

The quantitative results (Figure 3) for UTP24 h urine protein showed that, on D₂₉ after administration of the drug, the UTP24 h urine protein in the high dose group RX₋₁₂ (18.97 mg/DL) was less than the test values in the negative control group NOR (39.30 mg/DL), positive control group MET (39.65mg/DL), and RX₋₆ (43.33 mg/DL). The results demonstrated that, on D29 after administration of the drug, the drugs of the Invention may decrease significantly UTP24 h urine protein in mice.

### 2. Test for ALB urine albumin

Albumin (ALB) is a normal protein in blood and accounts for 60% of plasma total protein, with negative charge; molecular weight 69 KD and radius 3.6 nm. Normal glomerular basement membrane has filtration function, in a mean pore size of 5.5 nm, evenly with a layer of negative charge in the surface. Under normal conditions, ALB is difficult to pass through glomerular basement membrane, and only a little amount of ALB may be filtered; however, 95% ALB is reabsorbed in proximal convoluted tubule. Therefore, only a very little amount of albumin occurs in urine under physiological conditions. Albuminuria is a pathological state, in which serum albumin is present in urine. It is one of the types of proteinuria.

In the experiment, the ALB urine albumin content values are shown in Figure 4. According to the results, on D29 after administration of the drug, the ALB urine albumin content (0.1 g/L) in the high dose group RX₋₁₂ was significantly less than ALB urine albumin content in the negative control group NOR (0.3 g/L), positive control group MET (0.25 g/L), and RX-6 (0.33 g/L). The results demonstrated that, after D29 of raise, the drugs of the Invention may decrease significantly ALB urine albumin in mice.

### 3. Test for mAlb urine microalbumin

Microalbuminuria means occurrence of microalbumin in urine. Microalbunminuria (mAlb) means that the excretion rate of urine albumin is more than the normal range, but less than the urine protein level tested by conventional methods. mAlb urine microalbuminuria means occurrence of microalbumin in urine. Presently, it is considered that determination of urine microalbumin may reflect early nephropathy and kidney injury.

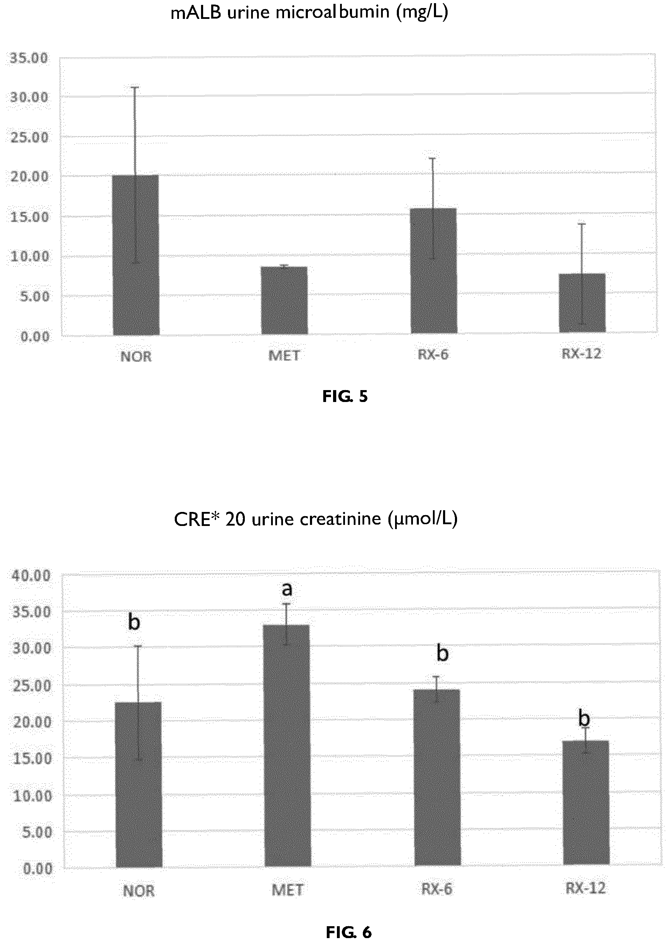

The mAlb urine microalbumin content values are shown in Figure 5. The mAlb urine microalbumin content in the high dose drug group RX₋₁₂ (7.37 mg/L) was less than that in the negative control group NOR (20.10 mg/L), positive control group MET (8.50 mg/L), and RX₋₆₍15.70 mg/L). The results demonstrated that, on D29 of raise, the drugs of the Invention may decrease mAlb urine microalbumin content in mice.

### 4. Test for urine creatinine

Urine creatinine is mainly originated from creatinine excreted in urine from blood after glomerular filtration.

According to Figure 6, the urine creatinine test results showed that, through 29-d raise, the urine creatinine in the high dose group RX-12 was decreased by 5.5 µmol/L than that in urine of mice in the negative control group NOR, while the urine creatinine in the positive control group MET was increased by 10.5 µmol/L than that in the negative control group NOR.

The results from the urine creatinine experiment part demonstrated that, after 29-d raise, the drugs of the Invention may decrease significantly urine creatinine value in mice.

### 5. Summary and Discussion

All the test results for urine total protein, urine albumin, urine microalbumin and urine creatinine showed that, the therapeutic effects of kidney in the high dose group RX-12 were better than those in the positive control group and negative control group. The results demonstrated that the high dose group of the Invention has effective therapeutic effects against nephropathy. What's more, many studies have demonstrated that, urine creatinine, urine microalbumin, and urine albumin are also risk factors and markers for retinopathy; according to the urine test results obtained by us, it may speculated that the drugs of the Invention may prevent, improve, or treat retinal microangiopathy.

### VI. Summary and conclusions of the animal experiment part

In this experiment part, a comparative experiment of the drugs of the Invention, and the positive control drug and negative control in body weight, blood glucose, and renal indices of mice was designed. The experimental results demonstrated that, compared with the positive control Metformin: Through 4-week raise, the drugs of the Invention may decrease significantly body weight of mice, decrease moderately and significantly blood glucose indices, and decrease a series of kidney test indices.

According to the results, it may judge that the drugs of the Invention have hypolipidemic, hypoglycemic, and kidney-injury-repairing therapeutic effects, and may also speculated that the drugs of the Invention may prevent, improve, or treat complications, e.g., retinal microangiopathy, and may also prevent, improve, or treat other various indices of metabolic syndrome and complications.

### Implementation Example 3: Clinical Trial Validation of TCM Complex

To investigate clinical use efficacy of the combinations in human body, patients with patients with diabetes mellitus were enrolled. Prior to administration of the drug, we conducted an incomplete statistics of the following indices of the patients, and also conducted a statistics of the indices 3-6 months later.

### I. Experimental Contents

### 1. Inclusion Criteria

The inclusion indices included: age, duration of disease, medication history, blood glucose value, vision, diabetic foot, nephropathy, fatty liver, blood pressure, etc.

Inclusion age ≥ 18 years and ≤ 75 years at enrollment, male or female.

Patients with the following conditions were not included:
1) Patients with active liver disease, including patients with alcoholic hepatitis, non-alcoholic fatty liver, viral hepatitis B, viral hepatitis C, chronic viral hepatitis, were not included temporarily into the statistics group as they require concurrently other TCM therapies;
2) Patients with preexisting advanced severe complications were not included temporarily into the statistics group, due to long term of treatment;
3) Patients with a disease or medical condition that may reduce life expectancy;
4) Patients treated previously with thiozolidinediones (TZD), human glucagon like peptide₋₁(GLP₋₁) drugs, or dipeptidyl peptidase IV inhibitors (DPP₋₄) in the previous 3 months;
5) Patients with uncontrollable hypoglycemia or hyperglycemia as judged (appropriate therapeutic effects could not be produced by dose titration, and hypoglycemia or hyperglycemia occurred recurrently; according to the guidelines for type I and type II diabetes mellitus, it is suggested that the low blood glucose reference value is ≤ 3.9 mmol/L, and the high blood glucose reference value is ≥ 16.7 mmol/L, for reference only);
6) Patients who were treated previously with systematic corticosteroids in the previous 2 months, or who were receiving systematic corticosteroids at screening (including glucocorticoids, but those who were administered topically within 7 d could be enrolled), immunosuppressants or cytotoxic therapy;
7) Patients suffered from the following heart disease in the previous 12 months are not included temporarily into the statistics group, as the required other concurrent TCM therapies:
   a. Decompensated cardiac insufficiency
   b. Unstable angina pectoris
   c. Cardiac infarction
8) Patients with uncontrollable through treatment/untreated severe hypertension (systolic blood pressure ≥ 180 mmHg and/or diastolic blood pressure ≥ 110 mmHg) were not included temporarily into the statistics group, as they required other concurrent TCM therapies;
9) Patients with a history of drug abuse in the previous 5 years;
10) Patients with moderate to severe anemia (hemoglobin ≤ 90 g/L) were not included temporarily into the statistics group as the required other concurrent TCM therapies;

### 2. Case

One hundred (100) patients with patients with diabetes mellitus were screened, of whom 51 patients, orally administered persistently with the prescription, were followed up. Those patients included 32 female patients and 20 male patients, aged between 20-75 years, mean 52.17 years: 20 patients with concurrent hypertension; 19 patients with concurrent nephropathy; 20 patients with concurrent hyperlipemia; 18 patients with concurrent diabetic oculopathy; and 14 patients with concurrent diabetic peripheral neuropathy.

### 3. Treatment Regimen

The patients in the treatment group were orally administered with the TCM products of the Invention; adult patients, 10-30 g, po, bid; one course of treatment consisted of 90 d.

### 4. Diagnostic Criteria

### 1) Criteria for judgment of patients with hyperglycemia

According to the diagnostic criteria of the American Diabetes Association (ADA), including: The fasting blood glucose (FPG) level is 126 mg/DL (7.0 mmol/L) or higher, or; in the period of the 75 g oral glucose tolerance test (OGTT), 2-h blood glucose level is ≥ 200 mg/DL (11.1 mmol/L), or; in patients with typical hyperglycemia or hyperglycemic crisis, the random blood glucose is ≥ 200 mg/DL (11.1 mmol/L), or; glycosylated hemoglobin (HbA1c) level is ≥ 6.5%.

Effective: FBG < 8.3 mmol/L, 2-h blood glucose level < 10 mmol/L, or blood glucose is decreased by 10%-29% than that prior to treatment;
Ineffective: Blood glucose decrease does not comply with the criteria (Pang Guoming, et al, 2017).

### 2) Criteria for judgment of patients with hypertension

As guided by the 2017 Guideline for High Blood Pressure in Adults, hypertension means a condition as follows: clinic blood pressure measurement, 140/90 mmHg, home blood pressure measurement HBPM 135/85 mmHg, daytime ambulatory blood pressure monitoring ABPM 135/85 mmHg, nighttime ambulatory blood pressure monitoring ABPM 120/70 mmHg, and 24-h ambulatory blood pressure monitoring ABPM 130/80 mmHg (Melvyn Rubenfire, MD, FACC, 2018).

### Effective: Blood pressure measurement is decreased by 10-20%;

### Ineffective: Blood pressure decrease does not comply with the criteria.

### 3) Criteria for judgment of patients with nephropathy

According to Diabetic Nephropathy Guidelines 2019, the diagnostic criteria for nephropathy are provided below:

Persistent proteinuria (>300 mg/24 h or >200 µg/min) is a characteristic clinical syndrome, diagnosed for at least 2 times in an interval of 3-6 months (VecihiBatuman, MD, 2019).

Effective: Urine protein excretion rate is recovered to normal or is decreased by 30% than that prior to treatment, and the grading score for TCM symptoms and decrease after treatment are ≥ 1/3-2/3;

Ineffective: Urine protein excretion rate does not comply with the effectiveness criteria or is increased contrary, and TCM symptoms grading score and decrease after treatment are < 1/3.

### 4) Criteria for judgment of patients with diabetic oculopathy

It seems that all oculopathies occur in patients with diabetes mellitus, e.g., fundus hemangioma, fundus hemorrhage, dacryocystitis, glaucoma, cataract, vitreous opacities, optic atrophy, macular degeneration, and retinal detachment Moreover, the chance for oculopathy in patients with diabetes mellitus is obviously more than that in non-diabetic population.

Effective: DR grading effective according to Messidor databank, including: ① Retinopathy grade; ② Grade risk of macular edema;

Ineffective: DR Grade ineffective according to Messidor databank.

### 5) Criteria for judgment of patients with peripheral neuropathy

### Diabetic peripheral neuropathy (DPN) is highly latent; more than

50% of the patients have no clinical symptoms, whose pathological degree is generally inconsistent with occurrence and severity of symptoms, and DPN is a high risk factor for foot ulcer and gangrene leading to amputation. Peripheral neuropathy is scored in accordance with (Michigan Diabetic Neuropathy Score, MD).

Effective: Effective means that MDNS score is reduced by ≥10 points.

### Ineffective: Ineffective means that MDNS score is reduced by < 10 points.

### II. Test Results

The effects in the patients with the above-mentioned diseases, prior to and after treatment, were compared, as shown in Table 2:

| **Table 2** Effects of treatment | | | | | | |
|---|---|---|---|---|---|---|
| | Patients with diabetes mellitus | Patients with hypertension | Patients with nephropathy | Patients with hyperlipidemia | Patients with diabetic oculopathy | Diabetic peripheral autonomic neuropathy |
| Reciprocal | 51 | 20 | 19 | 20 | 18 | 14 |
| Effective (patients) | 45 | 16 | 19 | 15 | 18 | 12 |
| Ineffective (patients) | 6 | 4 | 0 | 5 | 0 | 2 |
| Effective Ratio (%) | 88.24 | 80.00 | 100.00 | 75.00 | 100.00 | 85.71 |

According to the data in Table 2, it is obvious that, the clinical therapeutic effects in patients of the treatment group showed that, the total effective rate in patients with diabetes mellitus was 88.24%; in the data, small change occurred in early blood glucose, due to too ill complications, e.g., diabetic nephropathy and fatty liver; the effective rate in patients with hypertension was 80% as hypertension may be divided into congenital hypertension and acquired hypertension, some patients with refractory hypertension symptoms were not relieved after oral administration of the drug of a short term; the effective rate in patients with hyperlipemia was 75%; the curative rates in patients with diabetic oculopathy and nephropathy patients were high, as the early treatment of the drugs of the Invention were aiming for relief of complications, and the complications of nephropathic oculopathy were first were relieved; while the curative rate in patients with diabetic peripheral neuropathy reached 85.71%, and 2 patients who were with patients with severe diabetic foot, and it would be effective through long-term oral administration of the drug.

### II. Introduction to the Cases

### [Trial Case 1 related with diabetes mellitus]

### Implementation Subject A: female (43 years)

### Drug product administered: Powder of Implementation Example 1

Dose: Oral administration of 30 g of the drug in the morning under fasting state.

Oral administration of the drug, and duration of disease: The patient was with diabetes mellitus, discovered in an examination in 2017; preprandial blood glucose 17 mmol/L. No other treatment modes were adopted; in Feb 2017, the patient was orally administered with the prescription disclosed in this Implementation Example.

Records for oral administration of the drug: The patient was orally administered with the prescription for more than 1 month, and the blood glucose was decreased to normal level. Later, the patient was orally administered intermittently with the prescription for 6 months for consolidation of the efficacy. The disease did not recur in 2 years.

### [Trial Case 2 related with diabetes mellitus]

### Subject B: male (44 years)

### Drug product administered: Powder of Implementation Example 1

Dose: Oral administration of 30 g of the drug in the morning under fasting state.

Oral administration of the drug, and duration of disease: The patient was with diabetes mellitus, discovered in an examination in 2016; preprandial blood glucose 12 mmol/L; the patient was orally administered Metformin 1 tablet (0.5 g), 2 doses per day, and received TCM therapy for many times, with poor effects. The patient was orally administered with the prescription disclosed in this Implementation Example since Dec 2017.

Records for oral administration of the drug: The patient was orally administered with the prescription; the symptoms were improved 10 d later, and the blood glucose was decreased to 5.7 mmol/L. The disease did not recur so far.

### [Trial Case 3 related with diabetes mellitus]

### Subject C: female (66 years)

Dose: Oral administration of 30 g of the drug prior to the breakfast and supper under fasting state.

Oral administration of the drug, and duration of disease: A 25-year history of diabetes mellitus; the patient was injected with insulin 32 U and was orally administered with Metformin 3 tablets (0.5 g/tablet); the blood glucose was maintained at 8-12 mmol/L.

Records for oral administration of the drug: In the first 2 months when the patient was orally administered with the prescription, the physical symptoms of blurred vision, poor sleep, and lower limb inconvenience, etc., were relieved, but the blood glucose remained basically unchanged. By Month 3, the blood glucose was decreased stably to 6 mmol/L. In Month 4, insulin was reduced by 2 U/d, and then by 1 U/d until complete discontinuation; the blood glucose value was 14 mmol/L. The patient continued oral administration of the drug until the blood glucose was decreased gradually to normal.

### [Trial Case related with concurrent diabetes mellitus and hypertension]

### Subject D: female (57 years)

### Drug product administered: Powder of Implementation Example 1

Dose: Oral administration of 30 g of the drug prior to the breakfast and supper under fasting state.

Oral administration of the drug, and duration of disease: > 30-year duration of hypertension; the patient was orally administered with Micardis (Telmisartan) 1 tablet (80 mg)/d; in 2016, a test showed preprandial blood glucose 7.0 mmol/L, postprandial blood glucose 9.6 mmol/L, and glycosylated hemoglobin 6.7%; the patient was a patient with diabetes mellitus of double high indices. No other treatment modes were adopted; the patient was orally administered with the prescription disclosed in this Implementation Example.

Records for oral administration of the drug: Soon after oral administration of the TCM prescription disclosed in this Implementation Example, the eye problems disappeared, food intake was normal, and the sleep was normal. One month after oral administration of the drug, the preprandial blood glucose was decreased to 5.9 mmd/L. The patient switched to intermittent oral administration, 3 months after oral administration of the TCM prescription disclosed in this Implementation Example. The disease did not recur in 3 years. The blood pressure was decreased to normal; the patient did not require again any hypotensive drug.

### [Trial case 1 related with concurrent diabetes mellitus and nephropathy]

### Subject E: female (42 years)

### Drug product administered: Powder of Implementation Example 1

Dose: Oral administration of 30 g of the drug prior to the breakfast and supper under fasting state.

Oral administration of the drug, and duration of disease: 10-year duration of diabetes mellitus; the patient was orally administered with Metformin 1 tablet (0.5 g), 2 doses per day; a test showed urine protein 150 mg/DL. The patient was orally administered with the prescription disclosed in this Implementation Example.

Records for oral administration of the drug: One month after oral administration of the TCM prescription disclosed in this Implementation Example, urine protein was decreased to 11 mg/DL and the dose of the Western drugs were reduced to half. Later the patient continued oral administration of TCM for 6 months, until all the western drugs were discontinued, and the blood glucose was recovered to normal.

### [Trial case 2 related with concurrent diabetes mellitus and nephropathy]

### Subject H: male (50 years)

### Drug product administered: Powder of Implementation Example 1

Dose: Oral administration of 30 g of the drug prior to the breakfast and supper under fasting state.

Oral administration of the drug, and duration of disease: 15-year duration of diabetes mellitus; the patient was orally administered with of Metformin 2 tables (0.5 g), 2 doses per day, and Acarbose 1 tablet (50 mg), 3 doses per day; a test showed urine protein 100 mg/DL.

Records for oral administration of the drug: Two months after oral administration of the TCM prescription disclosed in this Implementation Example, urine protein was decreased to 20mg/DL, no foam occurred in urine, and the dose of the Western drugs were reduced to half. Later the patient continued oral administration of TCM for 3 months, until the blood glucose was recovered to normal.

### [Trial Case related with concurrent diabetes mellitus and peripheral neuropathy]

### Subject I: male (70 years)

### Drug product administered: Powder of Implementation Example 1

Dose: Oral administration of 30 g of the drug prior to the breakfast and supper under fasting state.

Oral administration of the drug, and duration of disease: Diabetes mellitus of more than 20 years; the patient was injected with the daily dose of insulin; preprandial blood glucose 10.1 mmol/L; feet and legs cold, with black spot, Michigan score for diabetic peripheral neuropathy score 25 points. By the hospital, the patient was notified of amputation half a year later.

No other treatment modes were adopted; in Feb 2017, the patient was orally administered with the prescription disclosed in this Implementation Example.

Records for oral administration of the drug: Three months after oral administration of the TCM prescription disclosed this Implementation Example, diabetic foot symptoms disappeared; the patient was orally administered intermittently with the prescription for 6 months, until leg and feet color were recovered and booting sensation disappeared.

### III. Summary and Conclusions of the Clinical Trial Part

In the clinical trial of the part, the patients with diabetes mellitus were mainly included, as well as the patients with complications including diabetic nephropathy patients, patients with hyperlipemia, patients with diabetic oculopathy, and patients with diabetic peripheral neuropathy, etc. The results of the clinical trial demonstrated that, the prescription has the following advantages:
1) The drugs of the Invention may relieve clinically effectively relevant complications, including diabetic nephropathy, oculopathy, hepatopathy, and peripheral neuropathy, etc. Compared with other current drugs against diabetes mellitus, the combinations of the Invention have significant synergistic effects in treatment of diabetes mellitus, and can improve significantly the symptoms of diabetes mellitus and also delay evolution of diabetes mellitus and reduce occurrence of complications.
2) Compared with the current chemical therapeutic drugs and natural pure TCM products for treatment of diabetes mellitus, the combinations of the Invention are components of food origin, with adverse reactions and side effects decreased significantly, so as to increase both dosing compliance in patients with diabetes mellitus and patients' quality of life.
3) The current therapeutic drugs against diabetes mellitus have acceptable therapeutic effects in the initial period of diabetes mellitus; however, with treatment time, an obvious drug resistance problem occurs, and the therapeutic effects against diabetes mellitus are decreased. The TCM combinations of the Invention contain multiple drug components, multiple effect targets, and the combinations of the Invention are of comprehensive effects and better therapeutic effects, and can prevent, improve, and treat various indices of patients with metabolic syndrome and complications, effectively solve tolerance problem of therapeutic drugs against diabetes mellitus; the therapeutic effects against diabetes mellitus were not decreased with treatment time.

Then the manufacture implementation examples for the combinations of the Invention are described.

### < Manufacture Implementation Example 1 > Preparation of the Drug Products

### <1-1> Preparation of powder

The TCM components were weighed proportionally and mixed, and pulverized to produce a powder in accordance with the conventional method for preparation of powder.

### <1-2> Preparation of water extract

The TCM components were weighed proportionally and mixed, and were decocted in accordance with the TCM decoction method and concentrated.

### <1-3> Preparation of tablets 100 mg

Water extract 10 mg in Implementation Example <1-2>, sodium carboxymethyl starch 22.2 mg, total amount of microcrystalline cellulose 37 mg, pregelatinized starch 11.1 mg, 50% ethanol/10%PVPK30 20 mg, magnesium stearate 0.37 mg are mixed to prepare tablets using the conventional method for preparation of tablets.

### <1-4> Preparation of pills

Water extract 1 g in Implementation Example <1-2>, lactose 1.5 g, glycerin 0.5 g, xylitol 0.5 g are mixed to prepare pills using the conventional method for preparation of pills.

### <1-5> Preparation of granules

Water extract 150mg in Implementation Example <1-2>, soybean extract 50 mg, dextrose 200mg, starch 600 mg were mixed and were added with 100 mg of 30% ethanol. The mixture was dried at 60 °C, and the prepared granules were filled into the package.

### < Manufacture Implementation Example 2 > Preparation of Food

A food containing the extract of the Invention was prepared as described below.

### <2-1> Preparation of flour food

The flour was added with 0.5-5.0 parts by weight of the water extract in Implementation Example <1-2>. In accordance with the conventional method, a flour mixture was used to prepare flour food, e.g., bread, cake, cookies, pancake, noodles, and chewsticks.

### <2-2> Preparation of milk products

Milk was added with 5-10 parts by weight of the water extract in Implementation Example <1-2>. In accordance with the conventional method, a milk mixture was used to prepare wholesome milk products, e.g., butter and ice cream.

### <2-3> Preparation of thick congee, a food supplement

The coarse grains, e.g., quinoa, barley, sticky rice, and black sesame, were stir-fried and gelatinized in accordance with the conventional method, and dried and pulverized to produce a 60-mesh powder.

The water extract in Implementation Example <1-2> was concentrated in vacuum, spray-dried, and pulverized to produce a 60-mesh dried powder. The coarse grains and the dried powder of the extract in Implementation Example <1-2> were mixed to prepare a food supplement

### < Manufacture Implementation Example 3 > Preparation of drink

### <3-1> Preparation of health tea

The tea was extracted at high temperature; the extract solution was filtered and then added with the following components and mixed; the extract was charged into a tank, sealed, and sterilized.

The Components include water extract 2g in Implementation Example <1-2>, citric acid 0.5 g, vitamin C 0.5 g, fructose-glucose syrup 60 g, red tea 5 g, and purified water added to 1000 g.

### <3-2> Preparation of meal replacement shake

The pulverized following components, or extract, were mixed to prepare a wholesome meal replacement shake: water extract 5g in Implementation Example <1-2>, konjac flour 20 g, complex vitamins 3 g, complex minerals 1 g, inulin 1 g, xylo-oligosaccharide 10 g, soy protein 10 g, and whey protein 10 g.

### <Manufacture Implementation Example 4 > Preparation of animal feed

### <4-1> Preparation of feed

Animal feed was prepared as described below, added to feed in 0.3%, dried adequately, and then dispensed.

### <1-1> Dregs mixture 95 g from preparation of powder

### Mold inhibitor 5 g, extracted from pure plant

### <4-2> Preparation of fermented feed additives

The following raw materials were mixed, charged into the fermentation bag, incubated under protection from light, emptied from the bag and then charged into the bag for 3-4 times, and dried and dispensed: <1-1> dregs mixture 50-500g from preparation of powder, rice bran or wheat bran 1000 g, raw sugar 3 g, enzyme microorganism 4 g, shell powder 50 g, and water 25%.

### <Manufacture Implementation Example 5> Preparation of veterinary drugs

Decomposed TCM mixture dregs in <4-2> were placed into an alkaline complex solution; the supernatant was transferred, concentrated, and dried to produce finished product containing humic acid in a content of ≥ 50%.

### Industrial Applicability

As described in the above text, the TCM extract or parts of the Invention are natural products and are effective for prevention, treatment, or delay of metabolic syndrome and complications; therefore, the Invention may be used to manufacture of drugs for treatment, prevention, or delay of metabolic syndrome, or improvement of immunity of the organism, and to manufacture health products, health functional food, or functional feed or feed additives.

The above-mentioned implementation examples are good implementation mode of the Invention; however, the implementation modes of the Invention are not restricted by the above-mentioned implementation examples. Any other change, modification, replacement, combination, simplification that are not deviated from the spiritual essence and principle of the Invention should be equivalent replacement modes and are within the protection range of the Invention.

## Claims

1. A traditional Chinese medicine (TCM) addition and subtraction prescription for prevention and/or treatment of dysmetabolic syndrome and complications, comprising: *Poria,* Semen *Coicis,* Rhizoma Dioscoreae, Semen Euryales, and Semen *Nelumbinis.*

2. The TCM addition and subtraction prescription according to claim 1, wherein, the TCM addition and subtraction prescription includes any one or more of the *Endothelium Corneum Gigeriae Galli,* Cortex *Cinnamomi, Massa Medicata Fermentata,* Radix *Puerariae,* or *Paeonia Lactiflora;*
preferably, the *Paeonia Lactiflora* may be replaced by any of the plants containing paeoniflorin, albiflorin, hydroxy paeoniflorin, oxypaeoniflorin, benzoyl paeoniflorin, lactiflorin, paeonol, paeonocide, paeonoside, or paeoniflorinin, or any combination of them;
preferably, the Semen *Nelumbinis* is replaceable by Plumula *Nelumbinis* or any one or combination;
preferably, the *Endothelium Corneum Gigeriae Galli* is replaceable with any of Fructus *Crataegi,* Semen *Raphani,* Fructus *Hordei Germinatus,* Fructus *Setariae Germinatus, Massa Medicata Fermentata,* protease, or other components with digestive or antanemic function, or any combination of them;
preferably, protease includes pepsin and trypsin;
preferably, protease includes protease hydrolysate, protease polypeptides, or other enzymes or polypeptides with digestive or antanemic function.

3. The TCM addition and subtraction prescription according to any one of claims 1-2, wherein, each component of the TCM addition and subtraction prescription is in a weight of 1-100 g;
preferably, each component of TCM addition and subtraction prescriptionis in a weight of 1-20 g.

4. The TCM addition and subtraction prescription according to claim 1, wherein, preferably, the TCM addition and subtraction prescription further includes excipients;
preferably, a weight ratio of the *Poria,* Semen *Coicis,* Rhizoma *Dioscoreae,* Semen *Euryales,* and Semen *Nelumbinis* is 1:1:1:1:1.

5. A product comprising the TCM addition and subtraction prescription according to any one of claim 1-4, wherein
preferably, the product is a drug product, health product, food, feed, or feed additive; and more preferably, the drug product and health product is for human use.

6. The product according to claim 5, wherein, dosage forms of the products include: powder, paste, granules, pills, tablets, capsules, dissolved medicine, soft extract, decoction, or injection.

7. The product according to claim 5, wherein, the products include: pulverized material, water extract, or organic solvent extract of the raw material components, and residues after extraction of the raw material; the pulverized material of the raw material components include active entity of plant components, or a mixture of such active entities.

8. A method for preparing the product according to any one of claim 5-7, wherein, the preparation method of the powder is: all components of product are prepared into a powder by processing procedure, pulverization procedure, and blending procedure; the processing procedure, pulverization procedure, and blending procedure may be in any order.

9. A method for preparing the product according to any one of claim 5-7, wherein, the decoction is prepared by a conventional preparation method.

10. The method for preparing the product according to claim 9, wherein, the decoction is concentrated to produce an extract, dried and pulverized, and then is subject to any of the following steps:
a) being tableted to produce tablets;
b) preparation to produce capsules, pills, and paste;
c) granulation to produce granules and dissolved medicine.

11. An application, comprising:
(1) application of the TCM addition and subtraction prescription according to any one of claims 1-4, in prevention and/or treatment of dysmetabolic syndrome and complications;
(2) application of the described TCM addition and subtraction prescription according to any one of claims 5-7, in prevention and/or treatment of dysmetabolic syndrome and complications;
(3) application of the described TCM addition and subtraction prescription according to any one of claims 8-10, in prevention and/or treatment of dysmetabolic syndrome and complications;
preferably, the dysmetabolic syndrome and complications include insulin resistance syndrome, diabetes mellitus, hyperlipemia, fatty liver disease, obesity, atherosclerosis, arterial sclerosis, and hypertension, and complications.
